(19) **Europäisches Patentamt**

**European Patent Office**

**Office européen des brevets**

(11) Veröffentlichungsnummer: **0 273 276**
**A1**

(12) # EUROPÄISCHE PATENTANMELDUNG

(21) Anmeldenummer: **87118405.7**

(22) Anmeldetag: **11.12.87**

(51) Int. Cl.⁴: **A61B 6/00** , A61G 13/00

(30) Priorität: **22.12.86 DE 8634352 U**

(43) Veröffentlichungstag der Anmeldung:
**06.07.88 Patentblatt 88/27**

(84) Benannte Vertragsstaaten:
**DE FR GB NL**

(71) Anmelder: **Siemens Aktiengesellschaft Berlin
und München
Wittelsbacherplatz 2
D-8000 München 2(DE)**

(72) Erfinder: **Schäfer, Willi, Dipl.-Ing. (FH)
Genglerstrasse 20
D-8520 Erlangen(DE)**

(54) **Röntgendiagnostikgerät.**

(57) Die Erfindung betrifft ein Röntgendiagnostikgerät mit einem Patientenlagerungstisch (1) und einem relativ zu diesem verstellbaren Bedienkästchen (2). Das Bedienkästchen (2) ist an einem im Bereich des Fußendes (5) des Patientenlagerungstisches (1) befestigten Tragarm (6) angebracht und mittels des Tragarmes (6) parallel zu einer Längskante des Patientenlagerungstisches (1) verschiebbar.

FIG 1

# Röntgendiagnostikgerät

Die Erfindung betrifft ein Röntgendiagnostikgerät mit einem Patientenlagerungstisch und einem relativ zu diesem verstellbaren Bedienkästchen.

Ein derartiges Röntgendiagnostikgerät ist aus der FR-PS 22 55 038 bekannt. Bei diesem Röntgendiagnostikgerät ist das Bedienkästchen um eine horizontale Achse schwenkbar unter der Lagerungsplatte des Patientenlagerungstisches angeordnet und kann ausgehend von einer ersten Endlage, in der an ihm angebrachte Bedienelemente durch die Lagerungsplatte verdeckt sind, in eine zweite Endlage gebracht werden, in der die Bedienelemente frei zugänglich sind. Ein auf dem Patientenlagerungstisch befindlicher Patient ist für eine Bedienperson somit ohne Behinderung durch das Bedienkästchen zugänglich, solange dieses nicht benötigt wird und demzufolge unter die Lagerungsplatte geschwenkt sein kann. Muß dagegen das Bedienkästchen während der Handhabungen an dem Patienten zugänglich sein, kann die Bedienperson, sofern die Handhabungen im Bereich des Bedienkästchens stattfinden, dieses während der Handhabungen ausgeschwenkt lassen, so daß es zwar jederzeit zugänglich ist, jedoch den Zugang zum Patienten behindert, oder das Bedienkästchen immer nur dann ausschwenken, wenn die Betätigung eines Bedienelementes erforderlich ist, was zeitraubend ist. Finden die Handhabungen am Patienten dagegen außerhalb des Bereiches des Bedienkästchens statt, z.B. im Bereich des Kopfendes des Patientenlagerungstisches, kann das Bedienkästchen zwar ausgeschwenkt sein, ohne die Bedienperson zu behindern, jedoch befindet es sich dann außer Reichweite der Bedienperson, so daß sich diese zur Betätigung eines Bedienelementes jedesmal zu dem Bedienkästchen begeben muß. Die Arbeit mit dem bekannten Röntgendiagnostikgerät gestaltet sich für die Bedienperson somit unbequem und zeitraubend.

Der Erfindung liegt die Aufgabe zugrunde, ein Röntgendiagnostikgerät der eingangs genannten Art so auszubilden, daß sich das Bedienkästchen jederzeit in Reichweite einer Bedienperson befinden kann, ohne diese bei Handhabungen an einem auf dem Patientenlagerungstisch liegenden Patienten zu behindern.

Erfindungsgemäß wird diese Aufgabe dadurch gelöst, daß das Bedienkästchen an einem im Bereich des Fußendes des Patientenlagerungstisches befestigten Tragarm angebracht und mittels des Tragarmes parallel zu einer Längskante des Patientenlagerungstisches verschiebbar ist. Die Bedienperson kann somit unabhängig davon, von welcher Stelle am Patientenlagerungstisch aus sie Handhabungen an einem auf diesem liegenden Patienten vorzunehmen wünscht, das Bedienkästchen stets in eine ihr genehme Position bringen, in der es sich in ihrer Reichweite befindet, ohne eine Behinderung darzustellen. Eine Behinderung durch den Tragarm ist dadurch ausgeschlossen, daß dieser im Bereich des Fußendes des Patientenlagerungstisches befestigt ist und somit den Zugang zu dem Patientenlagerungstisch nicht einschränkt. Der Tragarm kann sowohl an einer an der Decke oder an dem Fußboden angebrachten Säule als auch an dem Patientenlagerungstisch selbst angebracht sein. Falls der Röntgenstrahler und der zu diesem gehörige Strahlenempfänger an einem den Patientenlagerungstisch umgreifenden C-Bogen angebracht sind, wird außerdem durch die Befestigung des Tragarmes bzw. die Anordnung der diesen tragenden Säule im Bereich des Fußendes des Patientenlagerungstisches eine Behinderung der Einstellbarkeit des C-Bogens vermieden.

Eine Bedienperson kann das das Bedienkästchen dann besonders leicht in eine ihr genehme Position bringen, wenn nach Varianten der Erfindung der Tragarm um eine im Bereich des Fußendes des Patientenlagerungstisches angeordnete vertikale Achse schwenkbar gelagert ist. Dabei kann der Tragarm derart ausge bildet und um die vertikale Achse derart schwenkbar sein, daß das Bedienkästchen wahlweise längs einer der Längskanten des Patientenlagerungstisches verstellbar ist, so daß sich das Bedienkästchen unabhängig davon, auf welcher Seite des Patientenlagerungstisches sich eine Bedienperson befindet, stets in deren Reichweite befinden kann. Wenn das Bedienkästchen an dem Tragarm - schwenkbar angebracht, der Tragarm teleskopartig ausziehbar und das Bedienkästchen relativ zu einer Lagerungsplatte des Patientenlagerungstisches höhenverstellbar ist, ist jeweils eine besonders variable Einstellbarkeit des Bedienkästchens gewährleistet.

Wenn nach einer Ausführungsform der Erfindung der Tragarm im Falle eines Patientenlagerungstisches mit höhenverstellbarer Lagerungsplatte mit dieser verbunden ist, ist gewährleistet, daß das Bedienkästchen auch beim Verstellen der Höhe der Lagerungsplatte stets eine von einer Bedienperson gewünschte Höhe relativ zu der Lagerungsplatte beibehält.

Eine nochmals größere Freizügigkeit bei der Positionierung des Bedienkästchens ist dann gegeben, wenn der Tragarm einen ersten und einen zweiten Armabschnitt aufweist, die an ihren einen Enden mittels eines Gelenkes um eine vertikale Gelenkachse schwenkbar miteinander verbunden sind, wobei der erste Armabschnitt mit seinem anderen Ende um die vertikale Achse schwenkbar ist, während der zweite Armabschnitt an seinem anderen Ende das Bedienkästchen trägt. Falls der Tragarm in diesem Falle teleskopartig ausziehbar ist, ist es von Vorteil, den zweiten Armabschnitt teleskopartig ausziehbar zu gestalten. Außerdem kann vorgesehen sein, daß der erste Armabschnitt eine Länge aufweist, die größer oder gleich der halben Breite der Lagerungsplatte ist, und ausgehend von einer Position, in der er parallel zur Längsachse des Patientenlagerungstisches von diesem weggerichtet ist, nach beiden Seiten um 90° um die die Längsachse des Patientenlagerungstisches schneidende vertikale Achse schwenkbar ist, und der zweite Armabschnitt ausgehend von einer Position, in der er parallel zu dem ersten Armabschnitt von diesem weggerichtet ist, nach beiden Seiten um mehr als 90° - schwenkbar ist. Es ist dann möglich, das Bedienkästchen durch eine geeignete Schwenkbewegung des Tragarmes von der einen Seite des Patientenlagerungstisches auf die andere zu bringen, so daß das Bedienkästchen mittels des Tragarmes zu beiden Seiten des Patientenlagerungstisches relativ zu diesem einstellbar ist.

Nach einer weiteren Variante der Erfindung ist das Bedienkästchen in eine am Fußende des Patientenlagerungstisches befindliche Parkposition verfahrbar, in der es beim Betten eines Patienten auf die Lagerungsplatte keine Behinderung darstellt.

Ausführungsbeispiele der Erfindung sind in den beigefügten Zeichnungen dargestellt. Es zeigen:

Fig. 1 ein erfindungsgemäßes Röntgendiagnostikgerät,

Fig. 2 eine Seitenansicht des Röntgendiagnostikgerätes nach Fig. 1, und

Fig. 3 eine Einzelheit eines erfindungsgemäßen Röntgendiagnostikgerätes in perspektivischer Darstellung.

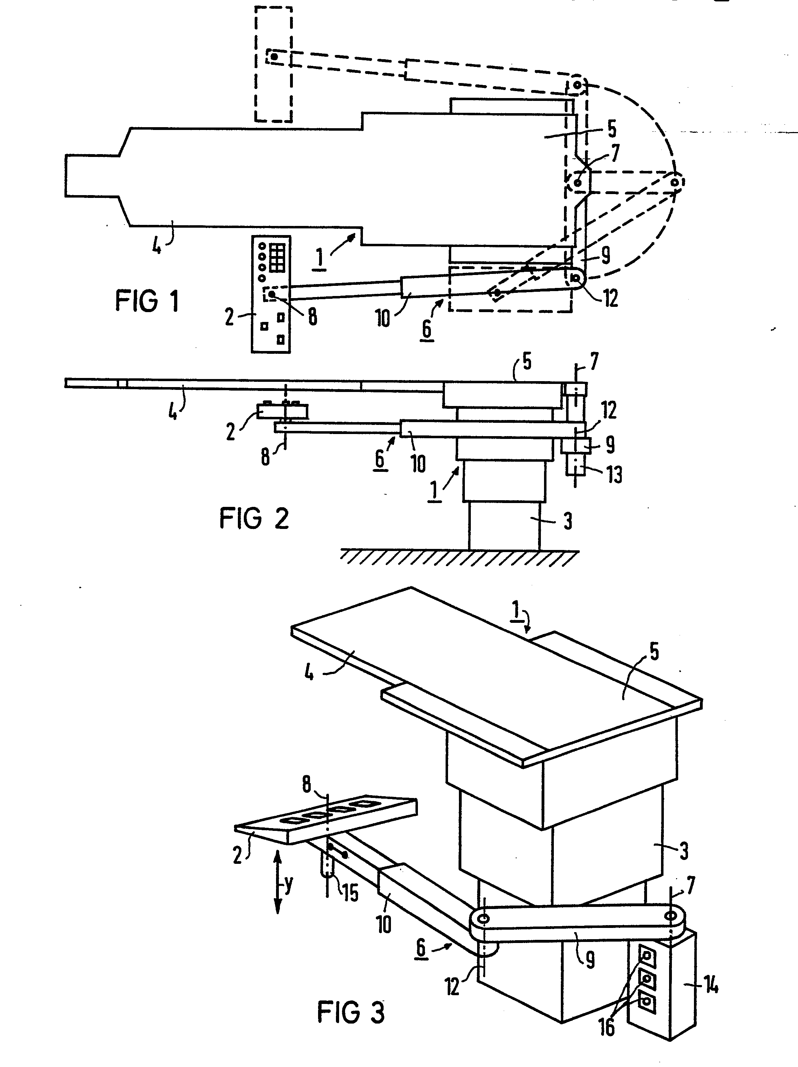

In den Fig. 1 und 2 sind von dem erfindungsgemäßen Röntgendiagnostikgerät nur der Patientenlagerungstisch 1 und das relativ zu diesem verstellbare Bedienkästchen 2 dargestellt. Der Patientenlagerungstisch 1 weist eine mittels eines teleskopartig ausfahrbaren Sockels 3 höhenverstellbare Lagerungsplatte 4 auf. Das Bedienkästchen 2 ist an einem im Bereich des Fußendes 5 des Patientenlagerungstisches 1 befestigten Tragarm 6 angebracht und mittels desselben 6 parallel zu einer Längskante der Lagerungsplatte 4 verschiebbar. Dabei ist der Tragarm 6 um eine im Bereich des Fußendes 5 des Patientenlagerungstisches 1 angeordnete vertikale Achse 7 - schwenkbar gelagert, während das Bedienkästchen 2 an dem Tragarm 6 um eine Achse 8 schwenkbar angebracht ist. Der Tragarm 6 weist einen ersten und einen zweiten Armabschnitt 9 bzw. 10 auf, die an ihren einen Enden mittels eines Gelenkes um eine vertikale Gelenkachse 12 schwenkbar miteinander verbunden sind. Der erste Armabschnitt 9 ist mit seinem anderen Ende um die vertikale Achse 7 schwenkbar, während der zweite Armabschnitt 10 an seinem anderen Ende das Bedienkästchen 2 trägt und teleskopartig ausziehbar ausgebildet ist.

Es wird somit deutlich, daß das Bedienkästchen 2 des erfindungsgemäßen Röntgendiagnostikgerätes von einer Bedienperson so positioniert werden kann, daß es sich unabhängig von der jeweiligen Position der Bedienperson am Patientenlagerungstisch 1 in deren Reichweite befindet und weder das Bedienkästchen 2 noch der Tragarm 6 die Bedienperson behindern. Da der Tragarm 6 bzw. dessen erster Armabschnitt 9, wie aus Fig. 2 ersichtlich, an der Lagerungsplatte 4 befestigt ist, folgt das Bedienkästchen 2 bei einer Verstellung der Höhe der Lagerungsplatte 4 deren Bewegung, so daß es sich unabhängig von der jeweils eingestellten Höhe der Lagerungsplatte 4 stets in Reichweite der Bedienperson befindet. Außerdem ist der Tragarm 6 und damit das Bedienkästchen 2 mittels eines Motors 13 in nicht näher dargestellter Weise, z.B. mittels eines Schraubentriebes, relativ zu der Lagerungsplatte 4 höhenverstellbar.

Wie aus den Fig. 1 und 2 ersichtlich ist, weist der erste Armabschnitt 9 eine Länge auf, die geringfügig größer als die Breite der Lagerungsplatte 4 ist. Er kann ausgehend von einer in Fig. 1 strichliert dargestellten Position, in der er parallel zur Längsachse des Patientenlagerungstisches 1 von diesem weggerichtet ist, nach beiden Seiten um 90° geschwenkt werden. Wie aus den Figuren weiter erkennbar ist, kann der zweite Armabschnitt 10 ausgehend von einer Position, in der er parallel zu dem ersten Armabschnitt 9 von diesem weggerichtet ist, nach beiden Seiten um mehr als 90° geschwenkt werden. Außerdem schneidet die vertikale Achse 7 die Längsachse des Patientenlagerungstisches 1. Es ist somit möglich, das Bedienkästchen 2 von der einen Seite des Patientenlagerungstisches 1 auf dessen andere Seite zu schwenken, wie dies in der Fig. 1 strichliert eingetragen ist. Eine Bedienperson kann somit, gleichgültig auf welcher Seite des Patientenlagerungstisches 1 sie sich befindet, das Bedienkästchen 2 in ihrer Reichweite positionieren.

Wie in der Fig. 1 außerdem strichliert eingetragen ist, kann das Bedienkästchen 2 beiderseits des Patientenlagerungstisches 1 in eine an dessen Fußende 5 befindliche Parkposition gebracht werden, wo es beim Betten eines Patienten auf die Lagerungsplatte 4 den Zugang zu dieser nicht behindert.

In der Fig. 3, in der gleiche Teile jeweils die gleichen Bezugsziffern wie in den Figuren 1 und 2 tragen, ist ein erfindungsgemäßes Röntgendiagnostikgerät dargestellt, das sich von dem zuvor beschriebenen dadurch unterscheidet, daß der das Bedienkästchen 2 tragende Tragarm 6 an einer im Bereich des Fußendes 5 des Patientenlagerungstisches 1 am Fußboden angebrachten Säule 14 um die vertikale Achse 7 schwenkbar angebracht ist. Der Tragarm 6 besteht wieder aus einem ersten Armabschnitt 9 und einem teleskopartig ausziehbaren zweiten Armabschnitt 10, wobei das Bedienkästchen 2 an dem zweiten Armabschnitt 10 mittels einer schematisch dargestellten Führung 15 in Richtung des Doppelpfeiles y höhenverstellbar angebracht ist, so daß eine Anpassung der Höhe des Bedienkästchens 2 an die Körpergröße einer Bedienperson bzw. die jeweilige Höhe der Lagerungsplatte 4 möglich ist.

An der Säule 14 sind Versorgungsanschlüsse 16 für Zusatzgeräte vorgesehen.

## Ansprüche

1. Röntgendiagnostikgerät mit einem Patientenlagerungstisch (1) und einem relativ zu diesem verstellbaren Bedienkästchen (2), **dadurch gekennzeichnet,** daß das Bedienkästchen (2) an einem im Bereich des Fußendes (5) des Patientenlagerungstisches (1) befestigten Tragarm (6) angebracht und mittels des Tragarmes (6) parallel zu einer Längskante des Patientenlagerungstisches (1) verschiebbar ist.

2. Röntgendiagnostikgerät nach Anspruch 1, **dadurch gekennzeichnet,** daß der Tragarm (6) um eine im Bereich des Fußendes (5) des Patientenlagerungstisches (1) angeordnete vertikale Achse (7) schwenkbar gelagert ist.

3. Röntgendiagnostikgerät nach Anspruch 2, **dadurch gekennzeichnet,** daß der Tragarm (6) derart ausgebildet und um die vertikale Achse (7) derart schwenkbar ist, daß das Bedienkästchen wahlweise längs einer der Längskanten des Patientenlagerungstisches (1) verstellbar ist.

4. Röntgendiagnostikgerät nach einem der Ansprüche 1 bis 3, **dadurch gekennzeichnet,** daß das Bedienkästchen (2) an dem Tragarm (6) - schwenkbar angebracht ist.

5. Röntgendiagnostikgerät nach einem der Ansprüche 1 bis 4, **dadurch gekennzeichnet,** daß der Tragarm (6) teleskopartig ausziehbar ist.

6. Röntgendiagnostikgerät nach einem der Ansprüche 1 bis 5, **dadurch gekennzeichnet,** daß das Bedienkästchen (2) relativ zu einer Lagerungsplatte (4) des Patientenlagerungstisches (1) höhenverstellbar ist.

7. Röntgendiagnostikgerät nach einem der Ansprüche 1 bis 6, wobei der Patientenlagerungstisch (1) eine höhenverstellbare Lagerungsplatte (4) aufweist, **dadurch gekennzeichnet,** daß der Tragarm (6) mit der Lagerungsplatte (4) verbunden ist.

8. Röntgendiagnostikgerät nach einem der Ansprüche 1 bis 7, **dadurch gekennzeichnet,** daß der Tragarm (6) einen ersten und einen zweiten Armabschnitt (9 bzw. 10) aufweist, die an ihren einen Enden mittels eines Gelenkes (11) um eine vertikale Gelenkachse (12) schwenkbar miteinander verbunden sind, wobei der erste Armabschnitt (9) mit seinem anderen Ende um eine vertikale Achse (7) schwenkbar ist, während der zweite Armabschnitt (10) an seinem anderen Ende das Bedienkästchen (2) trägt.

9. Röntgendiagnostikgerät nach den Ansprüchen 3 und 8, **dadurch gekennzeichnet,** daß der erste Armabschnitt (9) eine Länge aufweist, die größer oder gleich der halben Breite des Patientenlagerungstisches (1) ist, und ausgehend von einer Position, in der er parallel zur Längsachse des Patientenlagerungstisches (1) von diesem weggerichtet ist, nach beiden Seiten um 90° um die die Längsachse des Patientenlagerungstisches (1) schneidende vertikale Achse (7) schwenkbar ist, und der zweite Armabschnitt (10) ausgehend von einer Position, in der er parallel zu dem ersten Armabschnitt (9) von diesem weggerichtet ist, nach beiden Seiten um mehr als 90° schwenkbar ist.

10. Röntgendiagnostikgerät nach einem der Ansprüche 1 bis 9, **dadurch gekennzeichnet,** daß das Bedienkästchen (2) in eine am Fußende (5) des Patientenlagerungstisches (1) befindliche Parkposition verfahrbar ist.

86 P 3473  E

FIG 1

FIG 2

FIG 3

| | | | |
|---|---|---|---|
| | Europäisches Patentamt | **EUROPÄISCHER RECHERCHENBERICHT** | Nummer der Anmeldung |
| | | | EP 87 11 8405 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-3 137 516 (SIEMENS)<br>* Seite 4, Zeilen 4-6,19-21; Figur 1 *<br>--- | 1,2. | A 61 B 6/00<br>A 61 G 13/00 |
| Y | DE-U-8 521 246 (PICKER INTERNATIONAL)<br>* Seite 4, Zeilen 8-12; Seite 8, Zeilen 4-8; Seite 8, Zeile 34 - Seite 9, Zeile 5; Figuren 1,4 * | 1,2 | |
| A | | 6 | |
| A | FR-A-2 321 231 (SIEMENS)<br>* Seite 2, Zeile 25 - Seite 3, Zeile 18; Figuren 1-3 *<br>--- | 1-4,6-8 | |
| A | FR-A-1 295 849 (RITTER CO.)<br>* Seite 3, linke Spalte, Zeile 51 - rechte Spalte, Zeile 46; Seite 3, rechte Spalte, Zeile 56 - Seite 4, linke Spalte, Zeile 2; Figuren 1,2 *<br>--- | 2,6,7,9 ,10 | |
| A | FR-A-2 255 038 (SIEMENS)<br>* Seite 2, Zeile 29 - Seite 3, Zeile 4; Figuren 1,2 *<br>--- | 4 | **RECHERCHIERTE SACHGEBIETE (Int. Cl.4)** |
| A | CH-A- 347 604 (F. VAUCHER)<br>* Seite 1, Zeilen 1-9,23-29; Figuren *<br>----- | 5 | A 61 B<br>A 61 G |

Der vorliegende Recherchenbericht wurde für alle Patentansprüche erstellt

| Recherchenort | Abschlußdatum der Recherche | Prüfer |
|---|---|---|
| DEN HAAG | 04-03-1988 | FERRIGNO, A. |